# EUROPEAN PATENT APPLICATION

(11) **EP 3 984 575 A1**
(43) Date of publication of application: **20.04.2022**
(21) Application number: 20306206.2
(22) Date of filing: 14.10.2020
(51) Int. Cl.: A61M 5/315, A61M 5/178

(54) **END-OF-DOSE INDICATOR FOR MEDICAL INJECTION DEVICE**

(71) Applicant: Becton Dickinson France, 38800 Le Pont-de-Claix (FR)
(72) Inventor: DUBUC, Fabien, 38100 Grenoble (FR); GAGLIANO, Julien, 38240 Meylan (FR); PETIT, Louis, 38610 Venon (FR); DUCAROUGE, Pierre, 38100 Grenoble (FR)
(74) Representative: Germain Maureau

(57) **Abstract**

Provided herein is an end-of-dose indicator for a medical injection device including a housing having a proximal end, a distal end, and a sidewall therebetween defining an interior, the housing configured to be secured to a proximal end of a medical injection device. The indicator also includes a switch arranged within the housing interior and having an open position and a closed position, a circuit, and one or more indicators in electrical communication with the circuit. When in the closed position, the switch triggers the circuit to activate the indicator.

## Description

### BACKGROUND OF THE INVENTION

### Field of the Invention

The present disclosure relates generally to indicators for medical injection devices, and, in particular embodiments or aspects, to visual indicators providing an indication that a dose of a medicament has been completely delivered and the medical injection device can be removed from the site of injection.

### Description of Related Art

Medical injection devices often utilize indicators to inform users thereof that an injection has been completed. These indicators can take the form of audible, tactile, or visual indicators. However, in many cases the indicators are not sufficiently temporally delayed from the end of the dose, such that a user will remove the medical injection device from the site of injection before the entire dose of medicament has been delivered. Accordingly, there is a need in the art for a more robust indicator with a more reliable delay mechanism, such that full doses of a desired medicament is more reliably delivered.

### SUMMARY OF THE INVENTION

Provided herein is an end-of-dose indicator device for a medical injection device, having a housing having a proximal end, a distal end, and a sidewall therebetween defining an interior, the housing configured to be secured to a proximal end of a medical injection device, a switch arranged within the housing interior and having an open position and a closed position, a circuit, and one or more indicators in electrical communication with the circuit, wherein the switch, when in the closed position, triggers the circuit to activate the indicator.

Also provided herein is a medical injection system having a medical injection device including an injection device housing having a proximal end, a distal end, and a sidewall therebetween defining an injection device interior, a syringe received within the injection device interior, the syringe comprising a proximal end having a piston, a distal end having a needle, and a sidewall therebetween defining a syringe interior configured to receive a medicament, a plunger rod received within the injection device interior and configured to displace the piston distally to cause the medicament to be expelled through the needle, a drive assembly configured to displace the plunger rod distally, and a plunger rod follower having a distal end operatively coupled to the plunger rod and a proximal end;. The system also includes an end-of-dose indicator device having a housing having a proximal end, a distal end, and a sidewall therebetween defining an interior, the housing configured to be secured to a proximal end of a medical injection device, a switch arranged within the housing interior and having an open position and a closed position, a circuit, and one or more indicators in electrical communication with the circuit, wherein the plunger rod follower is configured such when as the plunger rod is displaced distally the proximal end of the plunger rod follower is displaced proximally and contacts the switch, moving the switch from the open position to the closed position and triggering the circuit to activate the indicator.

Further embodiments or aspects are provided in the following numbered clauses:
Clause 1. An end-of-dose indicator device for a medical injection device, comprising: a housing comprising a proximal end, a distal end, and a sidewall therebetween defining an interior, the housing configured to be secured to a proximal end of a medical injection device; a switch arranged within the housing interior and having an open position and a closed position; a circuit; and one or more indicators in electrical communication with the circuit, wherein the switch, when in the closed position, triggers the circuit to activate the indicator.
Clause 2. The end-of-dose indicator device according to clause 1, wherein the housing comprises a cylinder with a flared proximal end.
Clause 3. The end-of-dose indicator device according to clause 2, wherein the flared proximal end comprises a proximal-facing outer surface configured to receive a user's thumb.
Clause 4. The end-of-dose indicator device according to any of clauses 1-3, wherein the one or more indicators are one or more visual indicators.
Clause 5. The end-of-dose indicator device according to clause 4, wherein the one or more visual indicators are one or more light-emitting diodes.
Clause 6. The end-of-dose indicator device according to clause 4 or clause 5, wherein the housing further comprises one or more windows configured to allow the one or more visual indicators to be seen outside of the housing.
Clause 7. The end-of-dose indicator device according to clause 6, wherein the one or more windows are arranged at the distal end of the housing.
Clause 8. The end-of-dose indicator device according to clause 6, wherein the one or more windows are arranged at the proximal end of the housing.
Clause 9. The end-of-dose indicator device according to clause 6, wherein the one or more windows are arranged circumferentially around the housing.
Clause 10. The end-of-dose indicator device according to clause 9, wherein the one or more windows are arranged circumferentially around the distal end of the housing.
Clause 11. The end-of-dose indicator device according to clause 6, wherein the one or more windows are arranged along a longitudinal axis of the housing between the proximal end of the housing and the distal end of the housing.
Clause 12. The end-of-dose indicator device according to clause 6, wherein the one or more windows are arranged on a proximal-facing surface of the proximal end of the housing.
Clause 13. The end-of-dose indicator device according to any of clauses 1-12, wherein a shape of the housing matches a shape of the proximal end of the medical injection device.
Clause 14. The end-of-dose indicator device according to any of clauses 1-13, wherein the circuit is configured such that activation of the indicator is delayed following closure of the switch.
Clause 15. The end-of-dose indicator device according to clause 14, wherein activation of the indicator is delayed at least 2 seconds.
Clause 16. The end-of-dose indicator device according to clause 14, wherein activation of the indicator is delayed at least 5 seconds.
Clause 17. A medical injection system, comprising: a medical injection device comprising: an injection device housing having a proximal end, a distal end, and a sidewall therebetween defining an injection device interior; a syringe received within the injection device interior, the syringe comprising a proximal end comprising a piston, a distal end comprising a needle, and a sidewall therebetween defining a syringe interior configured to receive a medicament; a plunger rod received within the injection device interior and configured to displace the piston distally to cause the medicament to be expelled through the needle; a drive assembly configured to displace the plunger rod distally; a plunger rod follower comprising a distal end operatively coupled to the plunger rod and a proximal end; and an end-of-dose indicator device, comprising: an indicator housing comprising a proximal end, a distal end, and a sidewall therebetween defining an indicator interior, the indicator housing configured to be secured to the proximal end of the medical injection device; a switch arranged within the indicator housing interior and having an open position and a closed position; a circuit; and one or more indicators in electrical communication with the circuit, wherein the plunger rod follower is configured such when as the plunger rod is displaced distally the proximal end of the plunger rod follower is displaced proximally and contacts the switch, moving the switch from the open position to the closed position and triggering the circuit to activate the indicator.
Clause 18. The system according to clause 17, wherein the indicator housing comprises a cylinder with a flared proximal end.
Clause 19. The system according to clause 18, wherein the flared proximal end comprises a proximal-facing outer surface configured to receive a user's thumb.
Clause 20. The system according to any of clauses 17-19, wherein the one or more indicators are one or more visual indicators.
Clause 21. The system according to clause 20, wherein the one or more visual indicators are one or more light-emitting diodes.
Clause 22. The system according to clause 20, wherein the indicator housing further comprises one or more windows configured to allow the one or more visual indicators to be seen outside of the housing.
Clause 23. The system according to clause 22, wherein the one or more windows are arranged at the distal end of the indicator housing.
Clause 24. The system according to clause 22, wherein the one or more windows are arranged at the proximal end of the indicator housing.
Clause 25. The system according to clause 22, wherein the one or more windows are arranged circumferentially around the indicator housing.
Clause 26. The system according to clause 25, wherein the one or more windows are arranged circumferentially around the distal end of the indicator housing.
Clause 27. The system according to clause 22, wherein the one or more windows are arranged along a longitudinal axis of the indicator housing between the proximal end of the indicator housing and the distal end of the indicator housing.
Clause 28. The system according to clause 22, wherein the one or more windows are arranged on a proximal-facing surface of the proximal end of the indicator housing.
Clause 29. The system according to any of clauses 17-28, wherein a shape of the indicator housing matches a shape of the proximal end of the injection device housing
Clause 30. The system according to any of clauses 17-29, wherein the circuit is configured such activation of the indicator is delayed following closure of the switch.
Clause 31. The system according to clause 30, wherein activation of the indicator is delayed at least 2 seconds.
Clause 32. The system according to clause 30 or clause 31, wherein activation of the indicator is delayed at least 5 seconds.

### BRIEF DESCRIPTION OF THE DRAWINGS

**FIG. 1** is a perspective view of an end-of-dose indicator according to non-limiting embodiments or aspects described herein;
**FIG. 2** is a front view of an end-of-dose indicator according to non-limiting embodiments or aspects described herein;
**FIG. 3** is a side view of an end-of-dose indicator according to non-limiting embodiments or aspects described herein;
**FIG. 4** is a top view of an end-of-dose indicator according to non-limiting embodiments or aspects described herein;
**FIG. 5** is a bottom view of an end-of-dose indicator according to non-limiting embodiments or aspects described herein;
**FIG. 6** is a cross-sectional view along line 6-6 in **FIG. 4** of an end-of-dose indicator according to non-limiting embodiments or aspects described herein;
**FIG. 7** is a perspective view of an end-of-dose indicator according to non-limiting embodiments or aspects described herein;
**FIG. 8** is a perspective view of an end-of-dose indicator according to non-limiting embodiments or aspects described herein;
**FIG. 9** is a perspective view of an end-of-dose indicator according to non-limiting embodiments or aspects described herein;
**FIG. 10** is a side view of a system including an end-of-dose indicator according to non-limiting embodiments or aspects described herein;
**FIG. 11** is a top view of a system including an end-of-dose indicator according to non-limiting embodiments or aspects described herein;
**FIG. 12** is a cross-sectional view along line 12-12 in **FIG. 11** of a system including an end-of-dose indicator according to non-limiting embodiments or aspects described herein;
**FIG. 13** is a cross-sectional view along line 12-12 in **FIG. 11** of a system including an end-of-dose indicator according to non-limiting embodiments or aspects described herein; and
**FIG. 14** is a cross-sectional view along line 12-12 in **FIG. 11** of a system including an end-of-dose indicator according to non-limiting embodiments or aspects described herein.

### DESCRIPTION OF THE INVENTION

The use of numerical values in the various ranges specified in this application, unless expressly indicated otherwise, are stated as approximations as though the minimum and maximum values within the stated ranges are both preceded by the word "about". As used herein, the term "about" means the stated value ± 10%. In this manner, slight variations above and below the stated ranges can be used to achieve substantially the same results as values within the ranges. Also, unless indicated otherwise, the disclosure of these ranges is intended as a continuous range including every value between the minimum and maximum values. For definitions provided herein, those definitions refer to word forms, cognates and grammatical variants of those words or phrases.

The figures accompanying this application are representative in nature, and should not be construed as implying any particular scale or directionality, unless otherwise indicated. For purposes of the description hereinafter, the terms "upper", "lower", "right", "left", "vertical", "horizontal", "top", "bottom", "lateral", "longitudinal" and derivatives thereof shall relate to the invention as it is oriented in the drawing figures. However, it is to be understood that the invention may assume various alternative variations and step sequences, except where expressly specified to the contrary. Hence, specific dimensions and other physical characteristics related to the embodiments disclosed herein are not to be considered as limiting.

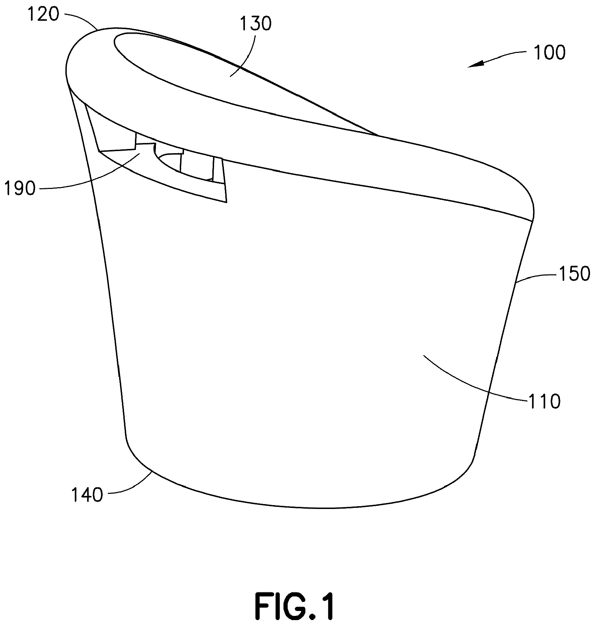

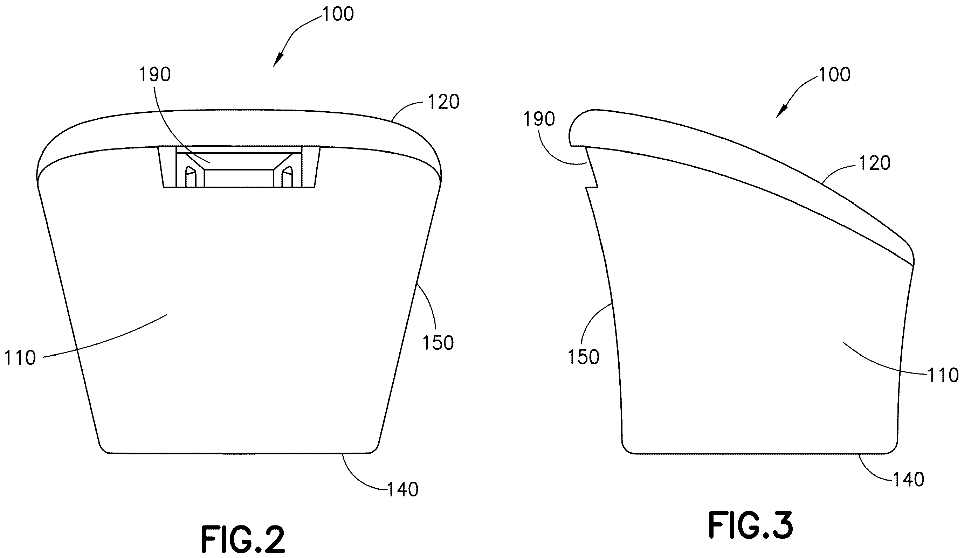

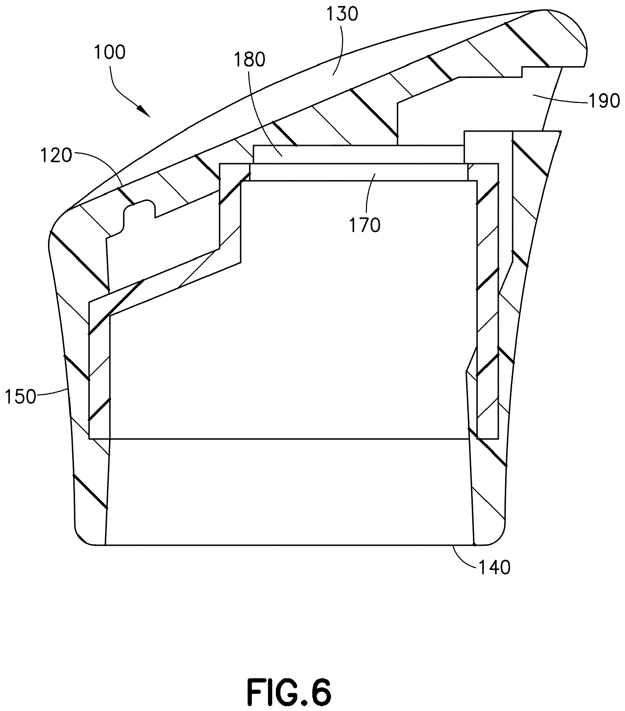

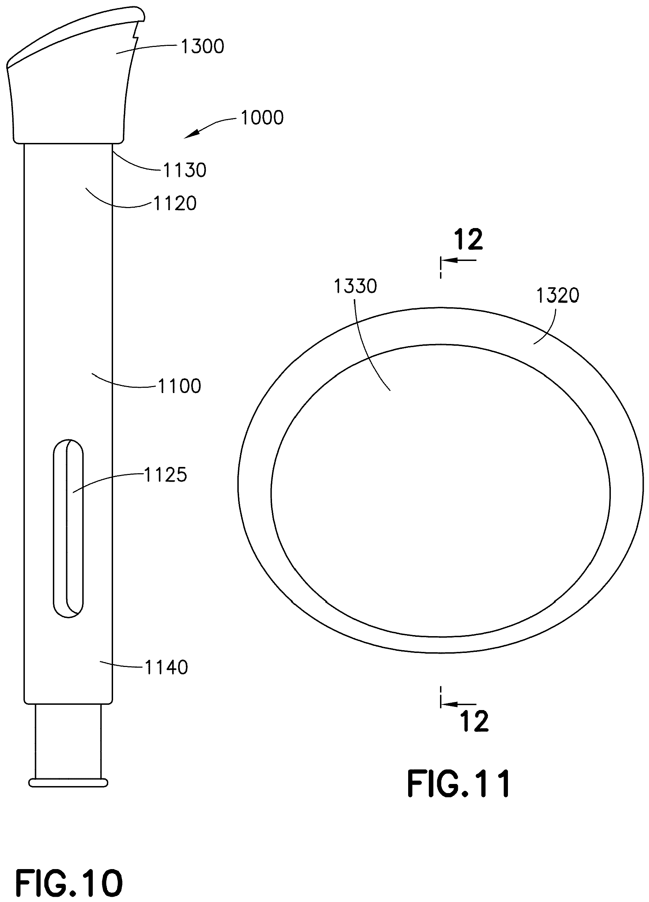

Turning to **FIGS. 1-6****,** provided herein is an end-of dose indicator device **100** for a medical injection device 1000, such as shown in **FIG. 11****.** End-of-dose indicator device **100** includes a housing **110** having a proximal end **120,** distal end **140,** and sidewall **150** therebetween defining an interior. As an end-of-dose indicator can, in non-limiting embodiments or aspects, be coupled to (reversible or permanently) a medical injection device (not shown), the interior of the housing **110** can, in non-limiting embodiments or aspects, be adapted to receive a portion of the medical injection device therein.

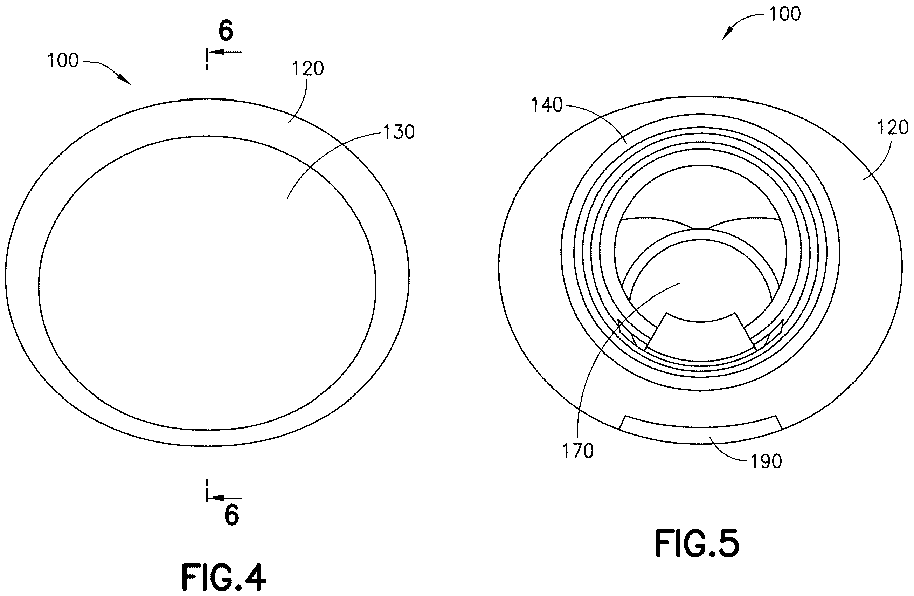

Housing **110** can assume any useful configuration to allow coupling to a medical injection device (not shown). In non-limiting embodiments or aspects, housing **110,** when viewed in cross-section from the proximal end **120** or distal end **140** thereof (e.g., **FIG. 4**), is substantially cylindrical, cylindrical, has an oval shape, and/or has the shape of an ellipse. In non-limiting embodiments or aspects, proximal end **120** is flared outward of the circumference of the sidewall **150.** In non-limiting embodiments or aspects, proximal end **120** has a proximal-facing outer surface **130.** Proximal-facing outer surface **130** can assume any suitable configuration, for example, to provide an ergonomic characteristic to the medical injection device and/or increase stability during the injection process. In non-limiting embodiments or aspects, proximal-facing outer surface **130** can be angled to allow for a thumb of a user of a medical injection device (not shown) to which end-of-dose injection device **100** is coupled to be placed thereon, for example, to provide a surface with which the user can apply force when applying the medical injection device a site of injection.

Housing **110** can be formed of any suitable material. In non-limiting embodiments, housing **110** is formed of polymeric materials, natural or synthetic rubber materials, metals, alloys, and/or combinations thereof. In non-limiting embodiments or aspects, proximal-facing outer surface **130** of housing **110** can include one or more resilient and/or elastomeric materials to provide an ergonomic feel, such as elastomeric polymers, synthetic and/or natural rubbers, foams, and combinations thereof.

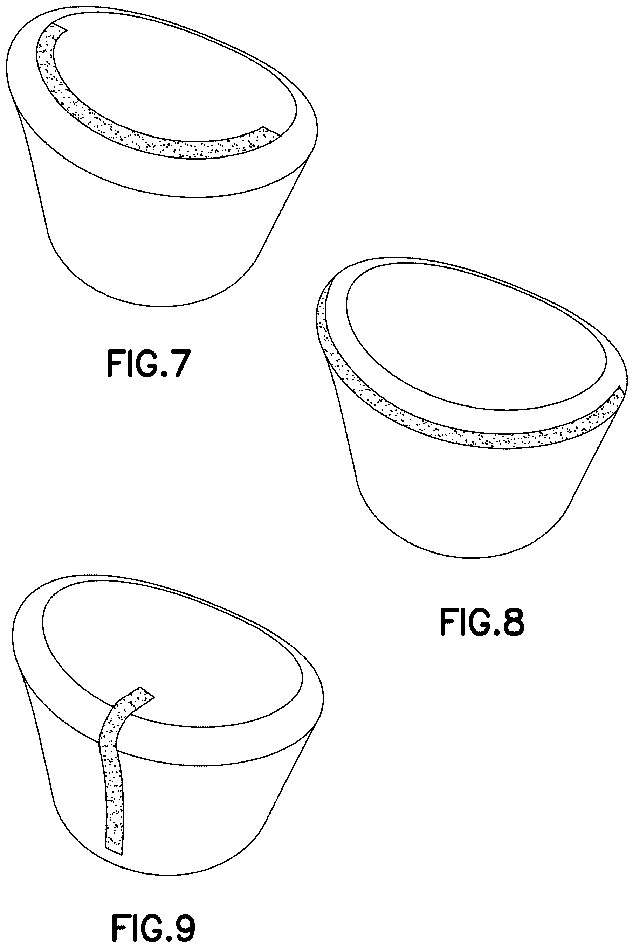

With continuing reference to **FIGS. 1-9****,** end-of-dose indicator device **100** includes a switch **160,** a circuit **170,** and an indicator **180** arranged within the interior of housing **110.** Switch **160** and circuit **170** can be arranged as is known in the art, such that switch **160** can assume an open configuration, in which current cannot pass through the circuit, and a closed configuration, in which current can pass through the circuit. End-of-dose indicator device **100** can include an electrochemical cell, such as a battery (not shown), in electrical communication with circuit **170.** Circuit **170** can include one or more batteries, wires, resistors, transistors, capacitors, fuses, speakers, haptic devices, and/or diodes. In non-limiting embodiments or aspects, indicator **180** is in electrical communication with and/or is part of circuit **170.** In non-limiting embodiments or aspects, indicator **180** is an audible indicator, a tactile indicator, and/or a visual indicator. In non-limiting embodiments or aspects, indicator **180** is a diode, such as a photodiode. In non-limiting embodiments or aspects, indicator **180** is a light-emitting diode (LED).

In non-limiting embodiments, circuit **170** is a delay circuit, in that circuit **170** is configured, for example, with one or more capacitors, such that there is a delay between closure of switch **160** and supply of power to indicator **180.** Such a delay provides an interval, for example, a pre-determined interval, between the end of delivery of medicament and the initiation of the indication. Those of skill in the art will appreciate that any suitable delay can be included based on basic circuity characteristics. In non-limiting aspects or embodiments, circuit **170** is configured to provide a delay between switch closure and initiation of the indication by at about 1, about 2, about 3, about 4, or about 5 seconds, all values and subranges therebetween inclusive.

In non-limiting embodiments or aspects, housing **110** can include one or more windows **190.** One or more windows **190** can be arranged on housing **110** in any desired configuration. One or more windows **190** can be one or more openings in housing **110,** and/or can include a translucent and/or transparent material that allows light to at least partially pass therethrough. In non-limiting embodiments or aspects, one or more windows **190** are arranged at proximal end **120,** distal end **140,** on proximal-facing outer surface **130** of housing **110,** longitudinally between proximal end **120** and distal end **140** of housing **110,** circumferentially about housing **110,** and/or any combination thereof. In non-limiting embodiments or aspects, windows **190** are arranged as shown in **FIGS. 1-3** and/or **7-9.**

With reference to **FIGS. 10-14****,** shown is a system **1000** including a medical injection device **1100** and an end-of-dose indicator device **1300** as described herein. While medical injection device **1100** is shown with minimal internal components, those of skill in the art will appreciate that any type of medical injection device, for example, those where the syringe **1170** is actively inserted at the injection site by a drive assembly **1250,** those where the syringe **1170** is retracted following delivery of the medicament **1230,** those where a button is used to deliver medicament **1230,** and those where no button is required (e.g., "push-on" activation) can be used with the end-of-dose indicator device described herein.

Medical injection device **1100** can include an injection device housing **1120** having a proximal end **1130,** a distal end **1140,** and a sidewall **1160** therebetween defining an interior. Injection device housing **1120** can be a two-piece housing, for example, a proximal part and a distal part that can be assembled. Injection device housing **1120** can also include a window **1125.**

Medical injection device **1100** can further include a syringe **1170** received in the interior thereof, and syringe **1170** can include a proximal end **1180,** a distal end **1200,** and a sidewall **1220** therebetween defining an interior configured to contain a composition, such as a medicament **1230.** Syringe **1170** can further include at distal end **1200** thereof a needle **1210** with one or more openings therein for delivery of medicament **1230.** Needle **1210** can be formed of any suitable material, and can be of any useful gauge. Syringe **1170** can further include at proximal end **1180** thereof a piston **1190.** Piston **1190** can be configured to seal the interior of syringe **1170,** and can be displaced distally to force medicament **1230** through needle **1210** to a site of injection. Piston **1190** can be formed of a natural rubber, a synthetic rubber, or mixtures thereof. In some aspects, the second component is formed of one or more of butyl rubber (including copolymers of isobutylene (about 97-98%) and isoprene (about 2-3%)), chlorinated butyl rubber, brominated butyl rubber, bromides of an isobutylene-paramethylstyrene copolymer, polyisoprene rubber, poly butadiene rubber, styrene ethylene butylene (SBS) rubber, styrene-isoprene (SIS) block polymers or styrene-isoprene/butadiene (SIBS), in which the content of styrene in the styrene block copolymer ranges from about 10% to about 70%, and preferably from about 20% to about 50%, epichlorohydrin rubber, styrene-butadiene (SBR) rubber, and mixtures thereof.

Syringe **1170** can be a pre-filled syringe, as is known in the art, and can include coatings, such as those including polydimethylsiloxane (PDMS) and/or other polymers, on sidewall **1220** and/or piston **1190** to reduce friction and/or reduce interactions between syringe **1170** components and compositions held within syringe **1170.** In non-limiting embodiments, syringe **1170** is a unitary syringe, and needle **1210** is formed with the syringe barrel. In non-limiting embodiments or aspects, syringe **1170** includes a needle hub, which holds needle **1210.** Syringe **1170** can be formed of any useful material, for example, plastic or glass, and piston **1190** can be formed of suitable materials such as natural and/or synthetic rubbers, polymeric materials, and the like.

In non-limiting embodiments or aspects, syringe **1170** can include a needle shield (not shown). In non-limiting embodiments or aspects, needle shield can be a one-piece needle shield formed of an elastomeric material, or a two-piece needle shield having an inner portion formed of an elastomeric material and an outer portion formed of a rigid material, such as a rigid polymeric material. Such needle shields are commonly referred to as rigid needle shields (RNS).

With continuing reference to **FIGS. 10-14****,** medical injection device **1100** can include plunger rod **1240** having a proximal end **1244** and a distal end **1246.** Plunger rod distal end **1246** can be configured to interact with piston **1190** of syringe **1170,** to displace piston **1190** distally to deliver medicament through needle **1210.** Medical injection device **1100** can also include a drive assembly **1250** configured to bias plunger rod **1240** distally. While drive assembly **1250** is exemplified in **FIGS. 10-14** as a spring coupled to plunger rod **1240,** those of skill in the art will appreciate that any suitable mechanism for displacing plunger rod **1240** distally can be used.

Medical injection device **1100** can further include a plunger rod follower **1260,** having a proximal end, a distal end, and a protrusion **1270** extending proximally from proximal end. Plunger rod follower **1260** can be coupled to or otherwise interact with proximal end **1244** of plunger rod **1240.** In non-limiting embodiments, plunger rod follower **1260** is configured to engage with plunger rod **1240** and injection device housing **1120** to cause plunger rod follower **1260** to move proximally once plunger rod **1240** has moved distally a certain amount. For example, and without limitation, proximal end **1244** of plunger rod **1240** can be received within an opening in plunger rod follower **1260,** applying a radial force and engaging plunger rod follower **1260** with an inner wall of injection device housing **1120** through a friction fit between plunger rod follower **1260** and injection device housing **1120** or through interaction of plunger rod follower **1260** and one or more protrusions **1270** extending inward from housing **1120.** This arrangement maintains proximal end of plunger rod follower spaced from proximal end **1130** of injection device housing and maintains protrusion **1270** within the interior of injection device housing **1120** and/or within opening **1150** in proximal end **1230** of injection device housing **1120.**

As plunger rod **1240** moves distally during delivery of a medicament **1230,** proximal end **1244** thereof is pulled out of engagement with plunger rod follower **1260,** allowing plunger rod follower **1260,** in non-limiting embodiments or aspects, to move radially inward and out of interaction with injection device housing **1120.** Arrangement of the interior of injection device housing **1120** at proximal end **1130** thereof can cause plunger rod follower **1260** to move proximally, and cause protrusion **1270** to pass through opening **1150** in proximal end **1230** of injection device housing **1120.** In non-limiting embodiments or aspects, movement of plunger rod follower **1260** proximally can cause plunger rod follower **1260** to contact proximal end **1130** of injection device housing **1100,** causing an audible and/or tactile indication. In non-limiting embodiments or aspects, no such indication is provided, so as to reduce confusion and remove a false indication that may cause the user to remove the injection device **1100** from the site of injection before the injection is completed.

System **1000** also can include an end-of dose indicator device **1300** as described above. End-of-dose indicator device **1300** includes a housing **1310** having a proximal end **1320,** distal end **1340,** and sidewall **1350** therebetween defining an interior. As shown in **FIGS. 10-14****,** end-of-dose indicator **1300** is coupled to (reversible or permanently) medical injection device **1100,** and the interior of the housing **1310** is adapted to receive a portion of the medical injection device **1100** therein. For example, and without limitation, end-of-dose indicator device **1300** can be a two-piece component that snaps together and surrounds proximal end **1130** of injection device housing **1120,** end-of-dose indicator device **1300** can be held on proximal end **1130** of injection device housing **1120** through a friction fit, a snap fit to a portion of indicator housing **1120,** or like known attachment mechanism. In non-limiting embodiments or aspects, indicator device is co-molded with injector device housing **1120,** and/or has a unitary construction with housing **1120.**

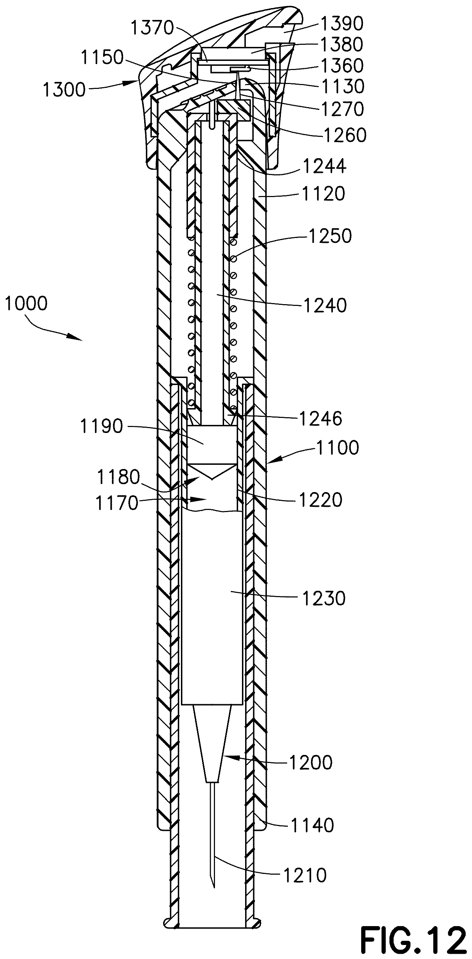

With continuing reference to **FIGS. 12-14****,** end-of-dose indicator device **1300** includes a switch **1360,** a circuit **1370,** and/or an indicator **1380,** each as described above, arranged within the interior of housing **1310.** In non-limiting embodiments or aspects, indicator **1380** is an audible indicator, a tactile indicator, and/or a visual indicator. In non-limiting embodiments or aspects, indicator **1380** is a diode, such as a photodiode. In non-limiting embodiments or aspects, indicator **1380** is an LED.

In non-limiting embodiments, circuit **1370** is a delay circuit as described above, in that circuit **1370** is configured, for example, with one or more capacitors, such that there is a delay between closure of switch **1360** and supply of power to indicator **1380.** In non-limiting aspects or embodiments as described above, circuit **1370** is configured to provide a delay between switch closure and initiation of the indication by at about 1, about 2, about 3, about 4, or about 5 seconds, all values and subranges therebetween inclusive.

In non-limiting embodiments or aspects, housing **1310** can include one or more windows **1390.** One or more windows **1390** can be arranged on housing **1310** in any desired configuration. One or more windows **1390** can be one or more openings in housing **1310,** and/or can include a translucent and/or transparent material that allows light to at least partially pass therethrough. In non-limiting embodiments or aspects, one or more windows **1390** are arranged at proximal end **1320,** distal end **1340,** on proximal-facing surface **1330** of housing **1310,** longitudinally between proximal end **1320** and distal end **1340** of housing **1310,** circumferentially about housing **1310,** and/or any combination thereof, for example, as described above and as shown in **FIGS. 1-3** and/or **7-9.**

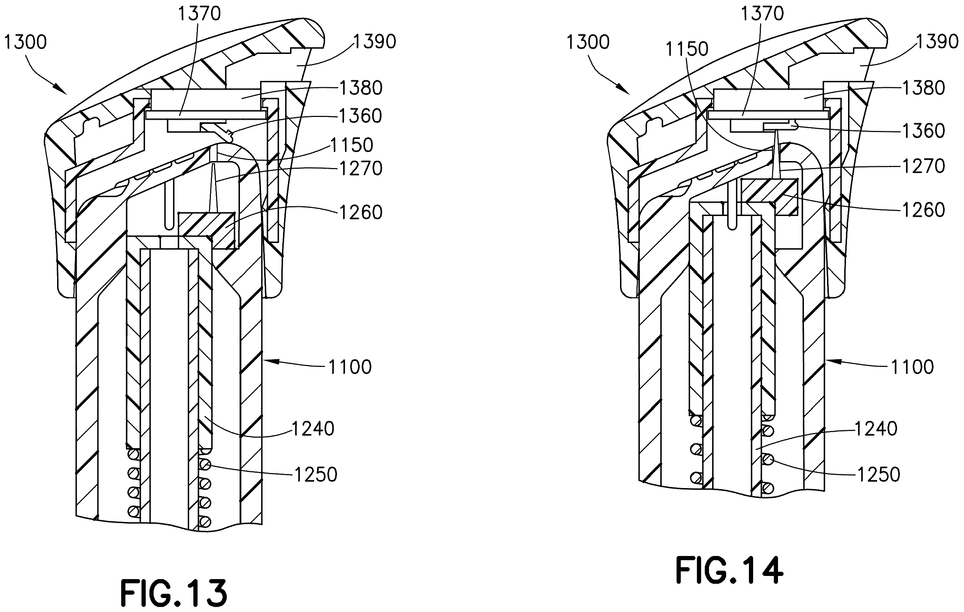

With reference to **FIGS. 13** and **14****,** shown is an enlarged view of the proximal end of system **1000** before/during plunger rod **1240** displacement and following displacement. As can be appreciated, through movement of plunger rod follower **1260** protrusion **1270** proximally, protrusion **1270** can pass through opening **1150** in proximal end **1230** of injection device housing **1120.** In doing so, protrusion **1270** can contact switch **1360** and move switch **1360** to a closed position, allowing current to accumulate/flow and for indicator **1380** to be activated.

In non-limiting embodiments or aspects, medical injection device **1100** can also include a removable cap (not shown). In non-limiting embodiments or aspects, removable cap can be configured to remove any needle shield, such as an RNS, that is included with syringe **1170.** In non-limiting embodiments or aspects, the cap is a two-piece cap, one portion of the two-piece cap (e.g., a cap remover) configured to be grasped by a used and pulled axially away from distal end **1140** of injection device housing and a second portion (e.g., a retainer) configured to grasp and remove a needle shield, such as an RNS (not shown).

Although the devices and systems have been described in detail for the purpose of illustration based on what is currently considered to be the most practical and preferred embodiments, it is to be understood that such detail is solely for that purpose and that the systems and methods are not limited to the disclosed embodiments, but on the contrary, is intended to cover modifications and equivalent arrangements that are within the spirit and scope of the appended claims. For example, it is to be understood that the present systems and methods contemplate that, to the extent possible, one or more features of any embodiment can be combined with one or more features of any other embodiment.

## Claims

1. An end-of-dose indicator device for a medical injection device, comprising:
a housing comprising a proximal end, a distal end, and a sidewall therebetween defining an interior, the housing configured to be secured to a proximal end of a medical injection device;
a switch arranged within the housing interior and having an open position and a closed position;
a circuit; and
one or more indicators in electrical communication with the circuit,
wherein the switch, when in the closed position, triggers the circuit to activate the indicator.

2. The end-of-dose indicator device according to claim 1, wherein the housing comprises a cylinder with a flared proximal end.

3. The end-of-dose indicator device according to claim 2, wherein the flared proximal end comprises a proximal-facing outer surface configured to receive a user's thumb.

4. The end-of-dose indicator device according to any of claims 1-3, wherein the one or more indicators are one or more visual indicators.

5. The end-of-dose indicator device according to claim 4, wherein the one or more visual indicators are one or more light-emitting diodes.

6. The end-of-dose indicator device according to claim 4 or claim 5, wherein the housing further comprises one or more windows configured to allow the one or more visual indicators to be seen outside of the housing.

7. The end-of-dose indicator device according to claim 6, wherein the one or more windows are arranged at the distal end of the housing.

8. The end-of-dose indicator device according to claim 6, wherein the one or more windows are arranged at the proximal end of the housing.

9. The end-of-dose indicator device according to claim 6, wherein the one or more windows are arranged circumferentially around the housing.

10. The end-of-dose indicator device according to claim 9, wherein the one or more windows are arranged circumferentially around the distal end of the housing.

11. The end-of-dose indicator device according to claim 6, wherein the one or more windows are arranged along a longitudinal axis of the housing between the proximal end of the housing and the distal end of the housing.

12. The end-of-dose indicator device according to claim 6, wherein the one or more windows are arranged on a proximal-facing surface of the proximal end of the housing.

13. The end-of-dose indicator device according to any of claims 1-12, wherein a shape of the housing matches a shape of the proximal end of the medical injection device.

14. The end-of-dose indicator device according to any of claims 1-13, wherein the circuit is configured such that activation of the indicator is delayed following closure of the switch.

15. The end-of-dose indicator device according to claim 14, wherein activation of the indicator is delayed at least 2 seconds.

16. The end-of-dose indicator device according to claim 14, wherein activation of the indicator is delayed at least 5 seconds.

17. A medical injection system, comprising:
a medical injection device comprising:
an injection device housing having a proximal end, a distal end, and a sidewall therebetween defining an injection device interior;
a syringe received within the injection device interior, the syringe comprising a proximal end comprising a piston, a distal end comprising a needle, and a sidewall therebetween defining a syringe interior configured to receive a medicament;
a plunger rod received within the injection device interior and configured to displace the piston distally to cause the medicament to be expelled through the needle;
a drive assembly configured to displace the plunger rod distally;
a plunger rod follower comprising a distal end operatively coupled to the plunger rod and a proximal end; and
an end-of-dose indicator device, comprising:
an indicator housing comprising a proximal end, a distal end, and a sidewall therebetween defining an indicator interior, the indicator housing configured to be secured to the proximal end of the medical injection device;
a switch arranged within the indicator housing interior and having an open position and a closed position;
a circuit; and
one or more indicators in electrical communication with the circuit,
wherein the plunger rod follower is configured such when as the plunger rod is displaced distally the proximal end of the plunger rod follower is displaced proximally and contacts the switch, moving the switch from the open position to the closed position and triggering the circuit to activate the indicator.

18. The system according to claim 17, wherein the indicator housing comprises a cylinder with a flared proximal end.

19. The system according to claim 18, wherein the flared proximal end comprises a proximal-facing outer surface configured to receive a user's thumb.

20. The system according to any of claims 17-19, wherein the one or more indicators are one or more visual indicators.

21. The system according to claim 20, wherein the one or more visual indicators are one or more light-emitting diodes.

22. The system according to claim 20, wherein the indicator housing further comprises one or more windows configured to allow the one or more visual indicators to be seen outside of the housing.

23. The system according to claim 22, wherein the one or more windows are arranged at the distal end of the indicator housing.

24. The system according to claim 22, wherein the one or more windows are arranged at the proximal end of the indicator housing.

25. The system according to claim 22, wherein the one or more windows are arranged circumferentially around the indicator housing.

26. The system according to claim 25, wherein the one or more windows are arranged circumferentially around the distal end of the indicator housing.

27. The system according to claim 22, wherein the one or more windows are arranged along a longitudinal axis of the indicator housing between the proximal end of the indicator housing and the distal end of the indicator housing.

28. The system according to claim 22, wherein the one or more windows are arranged on a proximal-facing surface of the proximal end of the indicator housing.

29. The system according to any of claims 17-28, wherein a shape of the indicator housing matches a shape of the proximal end of the injection device housing

30. The system according to any of claims 17-29, wherein the circuit is configured such activation of the indicator is delayed following closure of the switch.

31. The system according to claim 30, wherein activation of the indicator is delayed at least 2 seconds.

32. The system according to claim 30 or claim 31, wherein activation of the indicator is delayed at least 5 seconds.
